# FASCICULE DE BREVET EUROPEEN

(11) **EP 2 166 971 B1**
(45) Date de publication et mention de la délivrance du brevet: **08.08.2012**
(21) Numéro de dépôt: 08806141.1
(22) Date de dépôt: 01.07.2008
(51) Int. Cl.: A61B 17/70, A61B 17/064, A61B 17/80

(54) **DISPOSITIF DE PONTAGE POUR LAMINOPLASTIE ET SES APPLICATIONS**
ÜBERBRÜCKUNGSVORRICHTUNG FÜR DIE LAMINOPLASTIE UND ANWENDUNGEN DAVON
BRIDGING DEVICE FOR LAMINOPLASTY AND APPLICATIONS THEREOF

(30) Priorité: 03.07.2007 FR 0704800
(43) Date de publication de la demande: 31.03.2010
(73) Titulaire: Spineart SA, 1217 Meyrin (CH)
(72) Inventeur: MANGIONE, Paolo, F-33600 Pessac (FR); LEVIEUX, Jérôme, CH-1202 Genève (CH)
(74) Mandataire: Perin, Georges
(86) Numéro de dépôt international: PCT/FR2008/051217
(87) Numéro de publication internationale: WO 2009/007637

(56) Documents cités:
- WO-A-03/101319
- WO-A-2005/041752
- WO-A-2005/096969
- FR-A- 2 754 702
- JP-A- 10 179 622
- US-A1- 2002 156 477
- US-A1- 2003 125 738

## Description

La présente invention concerne une lame cervicale artificielle et ses applications.

La sténose cervicale est une pathologie qui fait parfois l'objet d'une intervention chirurgicale. La cervicarthrose en est l'étiologie principale, mais de nombreuses autres pathologies peuvent entraîner une réduction significative du diamètre du canal rachidien et une souffrance médullaire. Le but de l'intervention est alors d'augmenter les dimensions du canal médullaire.

Plusieurs techniques existent comme la laminectomie qui consiste en une ablation de la lame d'une vertèbre cervicale. Une autre est nommée la laminoplastie cervicale. Cette technique consiste à ouvrir la lame d'une vertèbre cervicale afin d'augmenter le volume du canal et à relier entre-elles les deux parties de la lame par un dispositif stable et fiable pour refermer le canal.

On connait un dispositif dans lequel les deux parties ouvertes et écartées de la lame sont reliées par un pont dont les deux extrémités en pointe sont insérées dans la partie spongieuse de la lame. Cette technique est convenable lorsque les lames sont suffisamment épaisses pour permettre l'installation du pont.

Ce type de dispositif présente cependant deux inconvénients majeurs. Lorsque la lame cervicale est très mince ce qui est fréquemment le cas, le pont ne peut pas pénétrer dans la partie spongieuse qui est quasi-inexistante. De plus, lors de l'écartement des deux parties de la lame sectionnée, il se peut qu'un des côtés se brise ou même les deux. Dans ce cas, la lame n'est plus tenue et la réalisation de la laminoplastie est impossible.

WO 2005/120367 décrit un dispositif de pontage ajustable en longueur pour laminoplastie. WO 2003/101319 décrit un dispositif de pontage comportant un système d'accrochage à une partie d'une lame.

US 2002/156477 se rapporte à un implant auto-maintenu pour fixer une couverture osseuse ou un fragment d'os à une ouverture dans le crâne. L'implant comporte un élément de support avec une face supérieure et une face inférieure face à la couverture osseuse. Disposée sur la face inférieure de l'élément de support on trouve une extension, portant une pointe de préférence parallèle à l'élément d'appui.

WO 2005/096969 A décrit un implant chirurgical pour stabiliser une lame vertébrale dans le cadre d'une opération de laminoplastie. Le dispositif comporte une entretoise longitudinale adaptée pour s'étendre en travers de la section entre les parties de lame et adapté pour être fixé à la lame.

FR-A-2 754 702 décrit un dispositif d'arthrodèse comportant une plaque équipée à chaque extrémité d'ancres destinées à être insérées dans des sièges préalablement percés dans les vertèbres à fixer. Après avoir été insérées, les d'ancres sont pliées l'une vers l'autre pour exercer une compression constante des vertèbres et assurer l'ancrage parfait.

US 2003/125738 décrit des dispositifs et les méthodes de fixation pour la stabilisation de la lame après laminoplastie. Le dispositif comporte une plaque avec plusieurs trous qui reçoivent des dispositifs de fixation à l'os.

Il serait donc souhaitable de disposer d'un pont polyvalent, convenant dans toutes les situations, notamment lorsque la lame est très fine et ne présente plus de zone spongieuse. Le dispositif devrait de préférence en outre permettre l'ancrage de la lame sur la vertèbre lorsqu'au moins un des côtés a été brisé lors de l'ouverture.

Après de longues recherches la demanderesse a mis au point un dispositif de pontage pour laminoplastie donnant satisfaction.

C'est pourquoi la présente demande a pour objet un dispositif de pontage pour laminoplastie comprenant un pont et deux bras de fixation tel que défini dans la revendication 1, notamment tel, que chaque bras de fixation comporte une languette terminée en pointe et munie d'aspérités anti recul, et qu'au moins un des bras de fixation comporte en outre un dispositif de blocage d'une lame cervicale pour pincer ladite lame cervicale entre la languette et ledit dispositif de blocage, les aspérités anti recul étant disposées entre ladite languette et ledit dispositif de blocage. La languette est terminée en pointe pour pouvoir être insérée dans l'épaisseur d'une lame cervicale.

Comme indiqué ci-dessous, le pincement est obtenu par déformation élastique.

L'ensemble du pont et des deux bras de fixation a habituellement une forme générale en U.

Un dispositif de pontage de l'invention peut comporter un dispositif de blocage d'un seul côté. De préférence, les deux bras de fixation comportent en outre un dispositif de blocage d'une lame cervicale pour pincer ladite lame cervicale.

Le dispositif de blocage ci-dessus est constitué d'une lamelle installée sensiblement parallèlement à la languette pour produire un pincement élastique de la lame cervicale. Le pincement se fait à la manière d'une pince d'accrochage de stylo ou à linge. Le pincement est obtenu par déformation élastique notamment de la première languette, du dispositif de blocage ou des deux.

Avantageusement cette lamelle comporte des aspérités prévues entre ladite languette et ladite lamelle, face aux aspérités anti recul prévues sur la languette terminée en pointe.

Avant mise en place du dispositif de blocage, un jeu par exemple de 1 à 7, ou de 2 à 5 mm peut exister entre ladite languette et ladite lamelle. Mais on peut ne prévoir aucun jeu entre la languette et la lamelle et une force de pression peut déjà être exercée par ces derniers l'un sur l'autre en raison de leur élasticité. Le jeu est celui entre l'extrémité des aspérités des languettes et des aspérités des lamelles les plus rapprochées.

Dans d'autres conditions préférentielles de mise en oeuvre de l'invention, le dispositif de blocage ci-dessus est constitué d'une lamelle courbée pour suivre le relief vertébral et de préférence suffisamment allongée pour comporter un orifice pour recevoir des vis prévues pour être fixées dans le massif articulaire de la vertèbre.

Les languettes terminées en pointe peuvent être insérées dans l'épaisseur de la lame cervicale. De plus ces languettes présentent des aspérités évitant qu'elles ne reculent une fois implantées. Le dispositif de blocage ou chaque dispositif de blocage coopère avec la languette pour former une pince du type pince accroche de stylo, afin de garantir la stabilité de l'ensemble sur la lame.

Dans le mode de réalisation dans lequel le dispositif de blocage est constitué d'une lamelle courbée, les orifices sont avantageusement conçus pour recevoir des vis montantes (de 30 à 50°).

Un dispositif de pontage pour laminoplastie selon l'invention peut être réalisé en deux pièces mais aussi en usinant une seule pièce formant l'ensemble.

En général, un dispositif de pontage selon l'invention sera réalisé en matériau métallique convenable pour une implantation. Il est de préférence réalisé en Titane ou alliage de Titane mais peut aussi être réalisé en résine plastique implantable comme le PEEK. Les éventuels différents éléments du dispositif de pontage sont avantageusement réalisés dans les mêmes matériaux.

Si l'on se réfère à une forme générale en U, la longueur du pont est avantageusement de 8 à 26, de préférence de 10 à 24, notamment de 12 à 22, tout particulièrement de 14 à 20 mm.

La longueur d'une languette est avantageusement de 3 à 13, de préférence de 4 à 12, notamment de 5 à 11, tout particulièrement de 6 à 10 mm.

La largeur d'une languette est avantageusement de 1 à 10, de préférence de 2 à 8, notamment de 2 à 6, tout particulièrement de 3 à 5 mm.

L'épaisseur d'une languette est avantageusement de 0,5 à 5, de préférence de 0,8 à 4, notamment de 1 à 3, tout particulièrement de 1 à 2 mm.

Les dimensions de la lamelle sont avantageusement du même ordre de grandeur que celles d'une languette.

Une lamelle courbée sera plus longue qu'une languette et en outre généralement plus large à son extrémité pour recevoir un passage de vis.

Les aspérités peuvent par exemple être en pointes de diamant ou en rainurages.

Les dispositifs de pontage objets de la présente invention possèdent de très intéressantes propriétés et qualités. Ils sont notamment capables d'être installés aisément grâce à leur fonction de pince et ce, que l'on insère la languette dans l'épaisseur de la lame ou que la languette soit située à l'intérieur de la lame lorsque celle-ci est trop étroite pour recevoir la languette. Le pincement par déformation élastique et non par serrage actif permet d'éviter l'utilisation d'un instrument de serrage qui rend le geste chirurgical difficile sur une lame souvent fragile et dans un zone très dangereuse car très proche de la moelle épinière. De plus, lorsque la lame a été brisée au moins d'un côté lors de l'intervention, la lamelle courbée longue de certains modèles possède des trous permettant le passage de vis à implanter dans les massifs articulaires, ce qui permet de refixer la lame à sa vertèbre.

Les dispositifs de pontage objets de la présente invention peuvent être utilisés comme suit : On ouvre tout d'abord la lame de la vertèbre cervicale en cause. Lorsque les lames sont suffisamment épaisses, on écarte les demi-lames, on plante les languettes terminées en pointe dans l'os spongieux de chaque demi-lame qui se trouve ainsi bloquée entre les languettes et le dispositif de blocage. Le canal est ainsi refermé.

Lorsque les lames ne sont pas suffisamment épaisses, on écarte les demi-lames, et on prend en sandwich chaque demi-lame entre la languette terminée en pointe et le dispositif de blocage. Le canal est ainsi refermé. Dans le mode de réalisation dans lequel le dispositif de blocage est constitué d'une seconde languette courbée, des vis de préférence montantes sont installées et fixées dans le massif articulaire de la vertèbre en passant par les orifices prévus à cette fin. Ce dernier mode de réalisation est particulièrement utile lorsqu'une au moins des demi lames a été cassée à sa base lors de l'écartement des demi lames. En effet dans ce cas la (ou les) lame(s) cassée(s) ne tient (tiennent) plus. Le système de pince maintient les demi-lames et le dispositif est accroché au massif articulaire, ce qui stabilise l'ensemble.

C'est pourquoi la présente demande a aussi pour objet un procédé de laminoplastie dans lequel on opère comme décrit ci-dessus.

Les conditions préférentielles de mise en oeuvre des dispositifs de blocage ci-dessus décrites s'appliquent également aux autres objets de l'invention visés ci-dessus, notamment aux procédés de laminoplastie précités.

L'invention sera mieux comprise si l'on se réfère aux dessins annexés sur lesquels
- la figure 1 représente une vue en perspective d'une vertèbre cervicale dont la lame a été sectionnée.
- la figure 2 représente une vue en perspective d'un dispositif de pontage selon un premier mode de réalisation.
- les figures 3 et 4 représentent un dispositif de pontage installé sur une vertèbre cervicale selon deux modes différents de montage.
- la figure 5 représente une vue en perspective d'un dispositif de pontage selon un second mode de réalisation, à fixation complémentaire par vis.

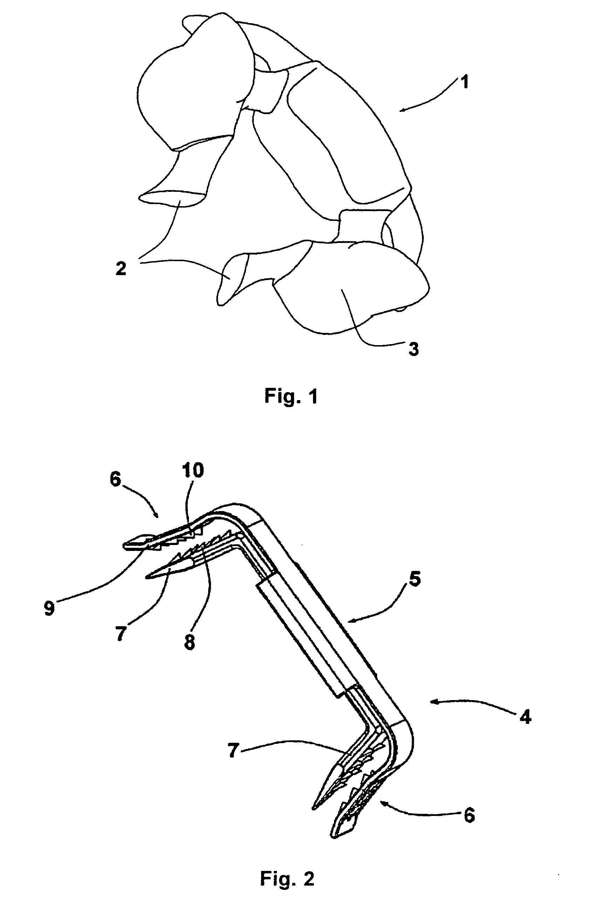

Sur la figure 1, on peut observer une vertèbre cervicale 1 dont la lame 2 reliant les deux massifs articulaires 3 a été sectionnée pour élargir le canal rachidien.

Sur la figure 2, est représenté un premier mode de réalisation d'un dispositif 4 de pontage selon l'invention. Celui qui est représenté a été réalisé en deux pièces distinctes ; mais un modèle élaboré en une seule pièce a également été fabriqué.

Ce dispositif 4 de pontage comprend dans ses grandes lignes un pont 5 allongé, généralement plat, qui comporte à chacune de ses extrémités un bras de fixation 6 installé environ perpendiculairement au pont 5, légèrement ouvert cependant comme représenté de telle sorte que les bras de fixation 6 ne sont pas parallèles.

Chacun des bras de fixation 6 du modèle représenté comprend d'une part une languette 7 et d'autre part un dispositif de blocage 9 qui, dans ce mode de réalisation, a la forme d'une lamelle.

Les languettes 7 ont une section généralement parallélépipédique aplatie et se terminent en pointe pour faciliter leur introduction dans l'os spongieux de la lame 2.

Les languettes 7 comportent des aspérités 8 conformées pour produire un effet anti-recul lorsque le dispositif de pontage 4 a été installé. Les aspérités 8 ont ici une forme de crochet.

Face à la languette 7 et à l'extérieur, le dispositif de blocage 9 est ici une lamelle 9 de forme et de section analogue à celle de la languette 7 sauf qu'elle ne se termine pas en pointe.

La lamelle 9 comporte également des aspérités anti-recul 10 face à celles 8 dont sont munies les languettes 7.

Un jeu d'environ 1 mm est prévu entre l'extrémité des aspérités 8 des languettes 7 et des aspérités 10 des lamelles 9.

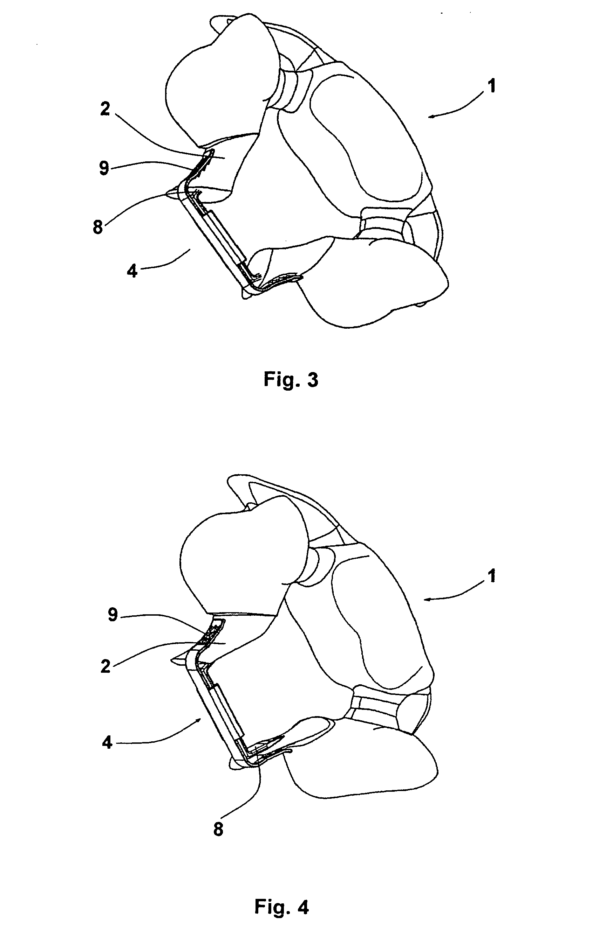

La figure 3 représente le montage d'un dispositif de pontage 4 selon l'invention sur une vertèbre cervicale 1.

La lame 2 a été sectionnée en son milieu et les deux demi-lames ont été ouvertes pour agrandir le canal.

Dans la mesure où la conformation de la lame le permettait, les languettes 7 ont été plantées dans les deux demi-lames 2.

La moitié de l'épaisseur de chaque demi-lame 2 se retrouve pincée entre les languettes 7 et les lamelles 9. Par écartement des languettes 7 et des lamelles 9 par rapport à leur position d'équilibre naturel, une pression s'exerce sur la lame 2. De plus les aspérités anti-recul 8 et 10 procurent une fixation efficace dans la vertèbre.

La vertèbre cervicale 1 de la figure 4 comporte une lame 2 de faible épaisseur.

Le montage de la figure 3 ne peut donc pas être mis en oeuvre. C'est là où la polyvalence du dispositif de l'invention entre en jeu.

On a pincé la totalité de l'épaisseur de la lame 2 entre chaque languette 7 et chaque lamelle 9 correspondante.

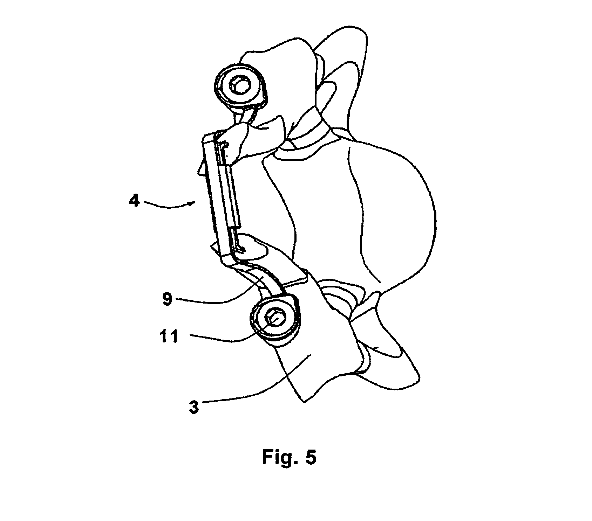

Sur la figure 5 est représentée une variante de réalisation. Non seulement, la lame ou une partie de la lame peut être pincée entre la languette 7 et la lamelle 9, mais en outre l'extrémité de chaque lamelle 9 comporte un orifice 11 permettant le passage d'une vis qui peut être fixée dans le massif particulaire 3. Un blocage encore plus efficace est ainsi obtenu. On peut noter que les trous sont conçus pour recevoir des vis montantes (de 30 à 50°).

## Revendications

1. Un dispositif de pontage pour laminoplastie comprenant un pont (5) et deux bras de fixation (7), chaque bras de fixation (7) comportant une languette munie d'aspérités (8) anti recul, et au moins un des bras de fixation (7) comportant en outre un dispositif de blocage (9) d'une lame cervicale (2) constitué d'une lamelle (9) installée sensiblement parallèlement à la languette pour produire un pincement élastique de ladite lame cervicale (2) entre la languette (7) et ledit dispositif de blocage (9) à la manière d'une pince d'accrochage de stylo, les aspérités (8) anti recul étant disposées entre ladite languette (7) et ledit dispositif de blocage (9), **caractérisé en ce que** chaque languette (7) est terminée en pointe de façon à pouvoir être insérée dans l'épaisseur d'une lame cervicale.

2. Un dispositif de pontage selon la revendication 1, **caractérisé en ce que** les deux bras de fixation (7) comportent en outre un dispositif de blocage (9) d'une lame cervicale (2) pour pincer ladite lame cervicale.

3. Un dispositif de pontage selon la revendication 1 ou 2, **caractérisé en ce que** la lamelle (9) comporte des aspérités (10) prévues entre la languette (7) et la lamelle (9), face aux aspérités anti recul (8) prévues sur la languette terminée en pointe.

4. Un dispositif de pontage selon l'une des revendications 1 à 3, **caractérisé en ce qu'**un jeu de 1 à 7 mm est prévu entre la languette (7) et la lamelle (9).

5. Un dispositif de pontage selon l'une des revendications 1 à 4, **caractérisé en ce que** le dispositif de blocage (9) est constitué d'une lamelle courbée (9) pour suivre le relief vertébral.

6. Un dispositif de pontage selon la revendication 5, **caractérisé en ce que** la lamelle courbée (9) est suffisamment allongée pour comporter un orifice pour recevoir des vis prévues pour être fixées dans le massif articulaire (3) de la vertèbre.

7. Un dispositif de pontage selon l'une des revendications 1 à 6, **caractérisé en ce qu'**il est réalisé en deux pièces.

8. Un dispositif de pontage selon l'une des revendications 1 à 6, **caractérisé en ce qu'**il est réalisé en une seule pièce.

9. Un dispositif de pontage selon l'une des revendications 1 à 7, **caractérisé en ce qu'**il a une forme générale en U, dont la longueur du pont (5) est de 10 à 24 mm, la longueur d'une languette (7) est de 4 à 12 mm, la largeur d'une languette (7) est de 2 à 8 mm et l'épaisseur d'une languette (7) est de 0,8 à 4 mm.

## Claims

1. A bridging device for laminoplasty comprising a bridge (5) and two fixing arms (7), each fixing arm (7) comprising a tongue having anti-pullout asperities (8), and at least one of the fixing arms (7) further comprising a cervical lamina (2) locking device (9) consisting of a slat (9) fitted approximately parallel to the tongue for clamping said cervical lamina (2) between the tongue (7) and said locking device (9) like the clip of a pen, the anti-pullout asperities (8) being located between said tongue (7) and said locking device (9), **characterized in that** each tongue (7) ends in a spike such as being able to be inserted into the thickness of a lamina.

2. A bridging device as claimed in claim 1, **characterized in that** both fixing arms (7) further comprise a cervical lamina (2) locking device (9) for clamping said cervical lamina.

3. A bridging device as claimed in claim 1 or 2, **characterized in that** the slat (9) comprises asperities (10) between the tongue (7) and the slat (9), facing the anti-pullout asperities (8) provided on the tongue that ends in a spike.

4. A bridging device as claimed in one of claims 1 - 3, **characterized in that** a gap of 1 to 7 mm is provided between the tongue (7) and the slat (9).

5. A bridging device as claimed in one of claims 1 - 4, **characterized in that** the locking device (9) consists of a slat (9) curved to follow the vertebral relief.

6. A bridging device as claimed in claim 5, **characterized in that** the curved slat (9) is long enough to contain a hole for a screw for fixing into the articular pillar (3) of the vertebra.

7. A bridging device as claimed in one of claims 1 - 6, **characterized in that** it is made in two parts.

8. A bridging device as claimed in one of claims 1 - 6, **characterized in that** it is made in one part.

9. A bridging device as claimed in one of claims 1 - 7, **characterized in that** its general shape is that of a U, in which the length of the bridge (5) is from 10 to 24 mm, the length of a tongue (7) is from 4 to 12 mm, the width of a tongue (7) is from 2 to 8 mm, and the thickness of a tongue (7) is from 0.8 to 4 mm.

## Patentansprüche

1. Überbrückungsvorrichtung für die Laminoplastie, umfassend eine Brücke (5) und zwei Befestigungsarme (7), wobei jeder Befestigungsarm (7) eine Lasche umfasst, die mit Oberflächenunebenheiten (8) zur Rückzugsicherung versehen ist, wobei mindestens einer der Befestigungsarme (7) ferner eine Feststellvorrichtung (9) für eine Zervikalscheibe (2) umfasst, die von einer Lamelle (9) gebildet ist, die im Wesentlichen parallel zur Lasche angeordnet wird, um ein elastisches Einklemmen der Zervikalscheibe (2) zwischen der Lasche (7) und der Feststellvorrichtung (9) in der Art einer Klammer zur Befestigung eines Stiftes hervorzurufen, wobei die Unebenheiten zur Rückzugsicherung (8) zwischen der Lasche (7) und der Feststellvorrichtung (9) angeordnet sind, **dadurch gekennzeichnet, dass** jede Lasche (7) mit einer Spitze endet, um in die Dicke einer Zervikalscheibe eingesetzt werden zu können.

2. Überbrückungsvorrichtung nach Anspruch 1, **dadurch gekennzeichnet, dass** die beiden Befestigungsarme (7) ferner eine Feststellvorrichtung (9) für eine Zervikalscheibe (2) umfassen, um die Zervikalscheibe einzuklemmen.

3. Überbrückungsvorrichtung nach Anspruch 1 oder 2, **dadurch gekennzeichnet, dass** die Lamelle (9) Oberflächenunebenheiten (10) umfasst, die zwischen der Lasche (7) und der Lamelle (9) gegenüber den Oberflächenunebenheiten zur Rückzugsicherung (8) vorgesehen sind, die auf der als Spitze endenden Lasche vorgesehen sind.

4. Überbrückungsvorrichtung nach einem der Ansprüche 1 bis 3, **dadurch gekennzeichnet, dass** ein Spiel von 1 bis 7 mm zwischen der Lasche (7) und der Lamelle (9) vorgesehen ist.

5. Überbrückungsvorrichtung nach einem der Ansprüche 1 bis 4, **dadurch gekennzeichnet, dass** die Feststellvorrichtung (9) von einer gekrümmten Lamelle (9) gebildet ist, um dem Wirbelrelief zu folgen.

6. Überbrückungsvorrichtung nach Anspruch 5, **dadurch gekennzeichnet, dass** die gekrümmte Lamelle (9) lang genug ist, um eine Öffnung zu umfassen, um Schrauben aufzunehmen, die dazu vorgesehen sind, im Gelenkmassiv (3) des Wirbels befestigt zu werden.

7. Überbrückungsvorrichtung nach einem der Ansprüche 1 bis 6, **dadurch gekennzeichnet, dass** sie aus zwei Teilen besteht.

8. Überbrückungsvorrichtung nach einem der Ansprüche 1 bis 6, **dadurch gekennzeichnet, dass** sie aus einem Teil besteht.

9. Überbrückungsvorrichtung nach einem der Ansprüche 1 bis 7, **dadurch gekennzeichnet, dass** sie eine allgemeine U-Form aufweist, wobei die Länge der Brücke (5) 10 bis 24 mm, die Länge einer Lasche (7) 4 bis 12 mm, die Breite einer Lasche (7) 2 bis 8 mm und die Dicke einer Lasche (7) 0,8 bis 4 mm beträgt.
